# EUROPÄISCHE PATENTSCHRIFT

(11) **EP 2 164 949 B1**
(45) Veröffentlichungstag und Bekanntmachung des Hinweises auf die Patenterteilung: **08.05.2013**
(21) Anmeldenummer: 08784689.5
(22) Anmeldetag: 10.07.2008
(51) Int. Cl.: C12N 5/00, A61L 27/22, A61L 27/38

(54) **BIOMATERIAL BASIEREND AUF EINEM HYDROPHILEN POLYMEREN TRÄGER**
BIOMATERIAL BASED ON A HYDROPHILIC POLYMER CARRIER
BIOMATÉRIAU À BASE D'UN SUPPORT POLYMÈRE HYDROPHILE

(30) Priorität: 13.07.2007 DE 102007034580
(43) Veröffentlichungstag der Anmeldung: 24.03.2010
(73) Patentinhaber: NMI Naturwissenschaftliches und Medizinisches Institut an der Universität Tübingen in Reutlingen Stiftung Bürgerlichen Rechts, 72770 Reutlingen (DE)
(72) Erfinder: WURST, Helmut, 77793 Pfullingen (DE)
(74) Vertreter: Witte, Weller & Partner
(86) Internationale Anmeldenummer: PCT/EP2008/005643
(87) Internationale Veröffentlichungsnummer: WO 2009/010232

(56) Entgegenhaltungen:
- EP-A- 0 844 252
- NIE T ET AL: "Production of heparin-functionalized hydrogels for the development of responsive and controlled growth factor delivery systems" JOURNAL OF CONTROLLED RELEASE - PROCEEDINGS OF THE THIRTHEENTH INTERNATIONAL SYMPOSIUM ON RECENT ADVANCES IN DRUG DELIVERY SYSTEMS 20071008 ELSEVIER NL, Bd. 122, Nr. 3, 10. Mai 2007 (2007-05-10), Seiten 287-296, XP022255961 online
- LÉVESQUE STÉPHANE G ET AL: "Synthesis of enzyme-degradable, peptide-cross-linked dextran hydrogels." BIOCONJUGATE CHEMISTRY 2007 MAY-JUN, Bd. 18, Nr. 3, Mai 2007 (2007-05), Seiten 874-885, XP002504996 ISSN: 1043-1802

## Beschreibung

Die vorliegende Erfindung betrifft ein Biomaterial für die Kultivierung von Zellen und/oder aus Zellen bestehendem Gewebe, basierend auf einem polymeren Träger, der zumindest ein vernetztes hydrophiles Polymer enthält.

Derartige Biomaterialien werden im Stand der Technik bspw. als sog. Drug-Release-Materialien, als Implantate, als Gewebe-bildende Matrizes oder als Materialien für die Kultur von Zellen eingesetzt, und oftmals auch als "Hydrogele" bezeichnet. Diese Biomaterialien werden üblicherweise für therapeutische Zwecke oder für die biologische Grundlagenforschung verwendet, da sie aufgrund ihrer einstellbaren biophysikalischen und biochemischen Eigenschaften wertvolle Werkzeuge in der regenerativen Medizin und im "Tissue Engineering", sowie bei der allgemeinen Kultivierung von Zellen darstellen.

Mit den Biomaterialien kann daher bspw. die Wiederherstellung der Struktur und Funktion von degeneriertem oder verletztem Gewebe erreicht werden, wenn sie mit Zellen beladen werden, anschließend implantiert werden und im Folgenden die Morphogenese von Gewebe somit fördern, oder wenn sie, nicht mit Zellen beladen, an Stellen implantiert werden, an denen sie durch das Einwachsen von Zellen und ggf. deren Differenzierung die Bildung von neuem Gewebe *in situ* fördern.

Wie bereits erwähnt, liegt ein weiteres Haupteinsatzgebiet von auf natürlichen oder synthetischen Materialien basierenden Biomaterialien in analytischen Zellkulturanwendungen, anhand derer bspw. die Wirkungsweise von Biofaktoren auf Zellen untersucht werden kann.

Im Stand der Technik sind verschiedene, auf natürlichen oder künstlichen Materialien basierende Biomaterialien mit verschiedenen Eigenschaften bekannt, die sich im Allgemeinen, und je nach erwünschtem Einsatzgebiet, durch eine hinreichende mechanische Stabilität, Elastizität, und durch eine Stabilität gegenüber einem Abbau auszeichnen, und die vor allem nicht toxisch sind. Häufig verwendete Materialien natürlichen Ursprungs sind Präparationen von Kollagen I. Dieses hydrophile Protein enthält Bereiche, die mit Zellen reagieren, wie bspw, Adhäsionssignale, Signale zur proteolytischen Spaltung vom Kollagen I, oder zur Zelldifferenzierung. Diese Signale und die dreidimensionale Natur der Kultivierung tragen dazu bei, dass sich Zellen in diesen Kollagen-Gelen natürlicher verhalten als in konventioneller zweidimensionaler Kultur auf Oberflächen von Kultivierungsbehältnissen.

Um die Funktionswege dieser Signale besser verstehen zu können, ist es notwendig, Biomaterialien einzusetzen, bei denen die Art und die Menge der Signalgruppen kontrolliert werden können, also ein Biomaterial, das die Zellen nicht schädigt und zunächst keine Signale trägt und somit Zellen gegenüber neutral ist, und das mit Signalgruppen modifiziert werden kann.

Im Stand der Technik (siehe bspw. Hersel et al., Biomaterials 24 (2003) 4385-4415; Lutolf und Hubbell, Nature Biotechnology, 23 (2005) 47-66) wird in aller Regel als ein solches zellneutrales Polymer Polyethylenglykol (im Folgenden öfters auch mit "PEG" abgekürzt) eingesetzt, da es nicht toxisch wirkt, nicht durch Zellen gebunden wird und hydrophil ist. Da lineares PEG jedoch nur an beiden Enden modifiziert werden kann, und da für die Bildung eines vernetzten Gels jedoch mindestens ein Reaktionspartner mindestens drei reaktive Gruppen tragen muss, werden einerseits zur Vernetzung Peptide eingesetzt, die mindestens drei reaktive Gruppen tragen, oder aber es wird verzweigtes PEG eingesetzt, das bspw. vier modifizierbare Enden besitzt.

Ferner offenbaren Nie et al. ("Production of heparin-functionalized hydrogels for the development of responsive and controlled growth factor delivery systems", Journal of Controlled Release 122 (2007) 287-296) ein dreidimensionales Hydrogel, das auf Heparin und PEG basiert ist.

Lévesque und Shoichit ("Synthesis of Enzyme-Degradable, Peptide-Cross-Linked Dextran Hydrogels", Bioconjugate Chem. 2007, 18, 874-885) offenbaren auf der anderen Seite ein auf Dextran basierendes Hydrogel.

Schließlich beschreiben Anseth et al. ("In situ forming degradable networks and their application in tissue engineering and drug delivery", Journal of Controlled Release 78 (2002) 199-209) die Herstellung eines dreidimensionalen Hydrogels, das auf dem Copolymer Polylaktat-Polyvinylalkohol basiert, das mit Acrylatgruppen funktionalisiert ist.

Die im Stand der Technik bekannten Polymere bzw. Hydrogele, haben jedoch den Nachteil, dass die Herstellung/Gewinnung der zur Vernetzung eingesetzten Peptide aufwändig und teuer ist, ebenso wie die Herstellung von verzweigtem PEG. Ferner ist von Nachteil, dass bei der Herstellung von Gelen aus Reaktionspartnern mit drei oder vier reaktiven Gruppen pro Molekül, sehr sorgfältig gearbeitet werden muss, da für die Gelbildung alle Reaktionspartner im richtigen Verhältnis stehen müssen.

Die im Stand der Technik bekannten Ansätze sind daher für die Entwicklung von Reagenzien für die Kultivierung von Zellen und Geweben von Nachteil.

Aufgabe der vorliegenden Erfindung ist es daher, ein Biomaterial bereitzustellen, mit dem die Nachteile der im Stand der Technik bekannten Biomaterialien überwunden werden können.

Diese Aufgabe wird durch die Bereitstellung eines Biomaterials gelöst, das auf einem polymeren Träger basiert, der zumindest ein vernetztes hydrophiles Polymer enthält, wobei das Polymer mit Maleimid-Gruppen funktionalisiert ist, und aus Polyvinylalkohol und Serumalbumin, oder Mischungen davon, ausgewählt ist.

Die der Erfindung zugrunde liegende Aufgabe wird auf diese Weise vollkommen gelöst.

Mit dem erfindungsgemäßen Biomaterial wird ein Polymer bereitgestellt, das sehr viele modifizierbare Gruppen aufweist, und das im Vergleich zu verzweigtem PEG gleichzeitig sehr kostengünstig herzustellen ist.

Mit dem erfindungsgemäßen Biomaterial können kostengünstig Hydrogele bereitgestellt werden, die einerseits bei der Kultivierung von Zellen, sowie deren Untersuchung hinsichtlich der Reaktion auf Biofaktoren eingesetzt werden können, oder aber andererseits auch ggf. bei der Kultivierung von Gewebe, sei es ex *vivo* durch Besiedelung mit Zellen und Implantation in ein zu regenerierendes Gewebe, oder *in situ* durch Bereitstellung einer Matrix, in welche Zellen einwandern können.

Darüber hinaus besteht die Möglichkeit, das Biomaterial bzw. das Hydrogel auch als "Drug-Release"-Biomaterial zu verwenden, also als Material, aus dem *in situ* Wirkstoffe freigesetzt werden. Solche Wirkstoffe können bspw. Arzneimittel, Pharmazeutika oder sonstige natürliche oder synthetische Wirkstoffe sein, also Substanzen, die eine bestimmte Wirkung auf das das Biomaterial umgebende Gewebe oder Zellen ausüben.

Vorliegend wird unter "Biomaterial" jedes natürliche und synthetische Material verstanden, das für die Herstellung von Matrizes oder Gelen geeignet ist, die für die Kultivierung von Zellen/Gewebe *ex vivo* oder *in vivo* verwendet werden können.

Unter "funktionalisiert" bzw. funktionalisieren ist vorliegend jeder (abgeschlossene) Vorgang zu verstehen, mit dem dem Polymer - bspw. durch Hinzufügen von Gruppen an das Polymer - eine Funktion verliehen wird, die es normalerweise nicht besitzt. Das derart "funkionalisierte Polymer" wird dann als "Biomaterial" bezeichnet.

Unter "Gewebe" wird vorliegend jeder Verband gleichartig differenzierter oder unterschiedlich differenzierter Zellen verstanden. In einem vielzelligen Organismus können Gewebe gemeinsam einen umschriebenen Aufgabenbereich erfüllen und/oder das spezifische Baumaterial für Organe bilden, gerade im Gegensatz zum bloßen Zusammenschluss undifferenzierter oder nur zeitweilig differenzierter Einzelzellen.

Mit "Kultivierung von Zellen oder Geweben" ist vorliegend jede Anwendung gemeint, bei der Zellen zum Zwecke der Vermehrung oder der Differenzierung, oder der Synthese von zellulären und extrazellulären Komponenten auf das Biomaterial aufgebracht werden bzw. einwandern können, und ggf. ein Gewebe bilden.

Das Biomaterial ist dabei zellneutral, was vorliegend einerseits wörtlich bedeutet, dass das Biomaterial sich gegenüber Zellen "neutral" verhält, und somit keinen Einfluss auf die Zellen hat, bzw. keine Reaktion bei den Zellen auslöst. Die Zellen binden bei diesen Materialien nicht "von sich aus" an das Polymer, sondern ggf. erst nach einer Modifizierung des Polymers mit entsprechenden Gruppen.

Das so bereitgestellte Biomaterial kann danach je nach Anwendung und Zweck gezielt mit Biofaktoren modifiziert werden. Zellen können in das Biomaterial eingebracht werden, mit den daran gekoppelten Biofaktoren interagieren und anschließend untersucht werden. Dadurch ist eine gezielte Untersuchung von bestimmten Biofaktoren bzw. deren Wirkung auf Zellen möglich.

Mit den im Stand der Technik bekannten, auf bspw. Kollagen basierenden Biomaterialien war dies nicht möglich, da diese bereits von sich aus mit den Zellen reagieren und Zellreaktionen auslösen können, was eine gezielte Untersuchung von Biofaktoren bzw. deren Wirkungsmechanismen auf Zellen unmöglich macht.

Die für die Funktionalisierung des Polymers eingesetzten Substanzen sind bei dem erfindungsgemäßen Biomaterial Maleimid-Gruppen. Über diese Gruppen können wiederum bspw. Biofaktoren an dem Polymer angebracht werden, an welche wiederum Zellen binden können. Das Polymer wird dann mit Zellen kombiniert und durch einen Vernetzer in ein dreidimensionales hydrophiles Netzwerk überführt. Andererseits können die Zellen aber auch, bspw. zur bloßen Kultivierung, auch ohne Biofaktoren in das Biomaterial eingebracht sein, so dass die Zellen bei dieser Ausführungsform direkt zu dem funktionalisierten Polymer gegeben werden, wonach dieses anschließend in Anwesenheit der Zellen vernetzt wird.

In den beschriebenen Versuchen konnte die Adhäsion von Zellen an Gelkomponenten und die Kultivierung von Zellen in dem Gel, sowie die Modifikation des Biomaterials mit Peptiden gezeigt werden.

Bei dem erfindungsgemäßen Biomaterial ist das Polymer aus Polyvinylalkohol (PVA), Serumalbumin (z. B. vom Rind), oder Mischungen davon ausgewählt.

Mit funktionalisiertem Polyvinylalkohol und funktionalisiertem Rinderserumalbumin konnten erfolgreich Versuche zur Kopplung von Peptiden an das Polymer, sowie zur Kultivierung von Zellen in dem Biomaterial gezeigt werden.

Der Einsatz der genannten hydrophilen, zellneutralen, funktionalisierten Polymere für die Kultivierung von Zellen und/oder Geweben ist im Stand der Technik nicht bekannt oder nahe gelegt. So wird zwar für die Antikörperherstellung maleimidfunktionalisiertes Rinderserumalbumin zur Kopplung von Peptiden an BSA eingesetzt, allerdings wurde dessen Einsatz zur Kultivierung von Zellen und/oder Geweben weder gezeigt noch vorgeschlagen.

Ferner ist im Stand der Technik der Einsatz von maleimidfunktionalisiertes Polyvinylalkohol bekannt (siehe Manecke und Vogt, Biochimie 62 (1980) 603-613), allerdings lediglich zur Immobilisierung von Enzymen, ohne einen Hinweis oder eine Offenbarung für einen Einsatz zur Zell-/Gewebekultur.

Dass maleimidfunktionalisierte Polymere, oder Polymere, die mit einer der anderen genannten Gruppen funktionalisiert werden, für einen Einsatz als Biomaterial zur Kultivierung von Zellen und/oder Geweben geeignet sind, war unerwartet und nicht nahe gelegt. Die der Anmeldung zugrunde liegenden Versuche zeigen zum ersten Mal, dass mit den erfindungsgemäßen Biomaterialien ein effizientes Werkzeug für die Zell-/Gewebekultur bereitgestellt wird, das herausragende Eigenschaften auch für die Untersuchung von Biofaktoren, bzw. für deren Reaktion auf/mit Zellen bietet.

Daher ist in weiteren Ausführungsformen bevorzugt, wenn das Biomaterial ferner zumindest einen Biofaktor aufweist, und insbesondere, wenn der Biofaktor zumindest eine Thiolgruppe aufweist, über welche der Biofaktor an Maleimidgruppen des Polymers kovalent gebunden ist.

"Biofaktor" soll vorliegend jede natürliche oder synthetische Substanz bedeuten, die einen Einfluss auf Zellen haben kann, bzw. die Reaktionen auf/in Zellen ausüben kann. Dieser Einfluss kann dabei auf bestimmte Zellen und bestimmte Bedingungen beschränkt sein, ohne dass die Substanz ihre Bedeutung als "Biofaktor" verlieren würde. Die chemische Beschaffenheit der vorliegend als "Biofaktoren" bezeichneten Substanzen ist dabei nicht auf eine bestimmte (Verbindungs-)Klasse beschränkt, sondern kann vielmehr jede natürliche und synthetische Substanz mit einschließen die von ihrer Natur aus und/oder in modifizierter Form irgendeine Wirkung auf biologische Zellen ausübt.

Unter "Signalgruppen" soll dabei vorliegend jeder Teil eines Biofaktoren verstanden werden, über welche eine Rekation von Zellen auf die Biofaktoren ausgelöst wird.

Mit "Zellen" sind dabei sämtliche Zellen umfasst, die mit dem vorliegenden Biomaterial kultiviert werden können, und schließen insbesondere eukaryontische Zellen, insbesondere Säugetierzellen wie humane und tierische Zellen mit ein.

Insbesondere ist bevorzugt, wenn der Biofaktor ausgewählt ist aus Peptiden, Proteinen, Nukleinsäuren, organische Wirkstoffe, Apoptose-induzierende Wirkstoffe, adhäsionsvermittelnde Wirkstoffe, wachstumshemmende Wirkstoffe, entzündungshemmende Wirkstoffe, Rezeptoragonisten- und Rezeptorantagonisten, oder Mischungen davon.

Diese Ausführungsform hat den Vorteil, dass gezielt verschiedene Faktoren ausgewählt werden können, deren Wirkung auf Zellen zwar bekannt ist, deren spezifische Wirkung in dreidimensionaler Kulturumgebung jedoch noch aufzudecken ist.

Insbesondere ist bevorzugt, wenn der Biofaktor ausgewählt ist aus: Proteinen der extrazellulären Matrix, Proteinen der Zelloberfläche, Antikörper, Wachstumsfaktoren, Zucker, Lektine, Kohlenhydrate, Zytokine, DNA, RNA, siRNA, Aptamere, sowie bindungs- oder wirkungsrelevanten Fragmenten davon, oder Mischungen davon.

Die Extrazelluläre Matrix (Extrazellularmatrix, Interzellularsubstanz, EZM, ECM) ist der Anteil des Gewebes, der von Zellen in den Interzellularraum, also den Raum zwischen den Zellen sezerniert wird. Die EZM stellt daher die Gesamtheit aller Makromoleküle dar, die sich außerhalb der Plasmamembran von Zellen in Geweben und Organen befinden. Die extrazelluläre Matrix besteht zu großen Teil aus verschiedenen Proteinen, insbesondere Kollagene, und Glykoproteinen, insbesondere Laminine, Vitronectin, Fibronectin, bzw. aus bestimmten Polysacchariden, den Glykosaminoglykanen.

Mit "bindungs- oder wirkungsrelevanten Fragmenten davon" sind Teile/Abschnitte der aufgeführten Biofaktoren gemeint, die, obwohl ev. nicht der vollständige Biofaktor eingesetzt wird, für sich genommen immer noch die gleiche/nahezu gleiche oder zumindest ähnliche Reaktion oder Wirkung auf Zellen ausüben, wie der komplette Biofaktor. Unter Reaktion kann dabei bereits die bloße Bindung von/an Zellen zu verstehen sein, aber auch die einer Bindung anschließende Reaktion in einer Zelle auf die Bindung, wie bspw. die Auslösung von bestimmten Reaktionswegen in den Zellen, die zu einer Produktion/Ausschüttung bestimmter Substanzen durch die Zellen führen können, oder aber zur einer Umwandlung oder Differenzierung der Zellen.

In diesem Zusammenhang ist bevorzugt, wenn das Peptid eine Sequenz aufweist, das ausgewählt ist aus SEQ ID Nr. 1 (Peptid "HW1"), SEQ ID Nr. 2 (Peptid "HW2") oder SEQ ID Nr. 9 (Peptid "HW9") aus dem beigefügten Sequenzprotokoll. Die Aminosäurensequenz 6-11 in HW 9 ist eine Adhäsionssequenz aus menschlichem Fibronectin, die aber auch in einigen anderen Organismen zu finden ist. Die flankierenden Sequenzen sind keines natürlichen Ursprungs.

Die Erfindung betrifft ferner ein Verfahren zur Herstellung eines Biomaterials für Zell-/Gewebekulturenanwendungen, das die folgenden Schritte aufweist:
a) Bereitstellen eines hydrophilen Polymers, das ausgewählt ist aus Polyvinylalkohol oder Serumalbumin, oder Mischungen davon,
b) Funktionalisieren des Polymers aus Schritt a) mit Maleimid und
c) Hinzufügen eines Vernetzers zum Vernetzen des in Schritt b) modifizierten Polymers.

Das erfindungsgemäße Verfahren hat den Vorteil, dass dadurch kostengünstig Biomaterialien hergestellt werden können, die Zellen gegenüber zellneutral sind, und mit welchen ein einfaches und zuverlässiges Werkzeug bereitgestellt wird, anhand dessen bspw. Zellen untersucht oder kultiviert werden können.

Insbesondere ist bei dem erfindungsgemäßen Verfahren bevorzugt, wenn es ferner den Schritt a)' aufweist:
a)' Aminierung des Polymers.

Dieses Verfahren wird insbesondere bei der Herstellung von maleimidfunktionalisiertem PVA eingesetzt, wohingegen bspw. BSA, d.h. Rinderserumalbumin, vor der Funktionalisierung nicht aminiert werden muss.

Ferner ist bevorzugt, wenn das Verfahren ferner nach Schritt b) den Schritt b)' aufweist:
b)' Hinzufügen von und Inkubieren mit Biofaktoren und/oder Zellen, insbesondere Säugetierzellen.

Bei dieser Ausführungsform werden vorteilhafterweise entweder zunächst Biofaktoren und anschließend Zellen zu dem funktionalisierten Polymer hinzugefügt, wodurch die Biofaktoren kovalent an die funktionellen Gruppen, und die Zellen an die Biofaktoren binden können. Andererseits besteht die Möglichkeit, dass die Zellen ohne kovalent gebundene Biofaktoren zu dem Polymer zugegeben werden, wodurch bspw. ein Biomaterial lediglich für die Kultivierung von Zellen, ohne eine Untersuchung der Reaktionen von Biofaktoren auf die Zellen.

Bei dem erfindungsgemäßen Verfahren ist das Polymer ausgewählt aus Polyvinylalkohol und Serumalbumin, insbesondere Rinderserumalbumin, oder Mischungen davon.

Diese Ausführungsformen haben den Vorteil, dass auf Substanzen zurückgegriffen werden kann, die sich nachgewiesenermaßen gegenüber Zellen neutral verhalten.

Ferner ist bei einer Ausführungsform des erfindungsgemäßen Verfahren bevorzugt, wenn der Biofaktor ausgewählt ist aus Peptiden, Proteinen, Nukleinsäuren, organische Wirkstoffe, Apoptose-induzierende Wirkstoffe, adhäsionsvermittelnde Wirkstoffe, wachstumshemmende Wirkstoffe, entzündungshemmende Wirkstoffe, Rezeptoragonisten- und Rezeptorantagonisten, und insbesondere aus Proteinen der extrazellulären Matrix, Proteinen der Zelloberfläche, Antikörper, Wachstumsfaktoren, Zucker, Lektine, Kohlenhydrate, Zytokine, DNA, RNA, siRNA, Aptamere, sowie bindungs-oder wirkungsrelevanten Fragmenten davon, oder Mischungen davon.

Bei einer Weiterbildung des erfindungsgemäßen Verfahrens ist bevorzugt, wenn der Vernetzer in Schritt c) ausgewählt ist aus Dithio-PEG oder SH-modifiziertem Dextran.

In der Erfindung zugrunde liegenden Versuchen konnte der Einsatz dieser Vernetzer erfolgreich gezeigt werden. Ferner ist von Vorteil, dass beide Substanzen im Stand der Technik hinreichend beschrieben und auf ev. unerwünscht Nebenreaktionen getestet sind.

Bei dem erfindungsgemäßen Verfahren ist dabei bevorzugt, wenn ein erfindungsgemäßes Biomaterial, wie beschrieben, hergestellt wird.

Vorliegend wird auch die Verwendung von Maleimid zur Funktionalisierung von hydrophilen Polymeren für die Herstellung eines Biomaterials für Zellkulturanwendungen beschrieben, und insbesondere die Verwendung von Maleimid zur Herstellung eines wie zuvor beschriebenen erfindungsgemäßen Biomaterials.

Es versteht sich, dass zur genauen Untersuchung und Analyse der Zellen, sollte diese gewünscht sein, das Biomaterial durch jeweils spezifisch geeignete Substanzen abgebaut werden kann. Dabei hängt die einzusetzende Substanz von dem verwendeten Vernetzer und/oder Polymer ab, so dass bspw. bei der Verwendung von Dextran als Vernetzer oder als Polymer Dextranasen zum Abbau eingesetzt werden können.

Weitere Vorteile ergeben sich aus der Beschreibung und der beigefügten Zeichnung.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: Die Ergebnisse von Adhäsionsversuchen von 3T3-Zellen an verschiedene Gelkomponenten;
- Fig. 2a, b: konfokale Laser Scanning Mikroskopie-Aufnahmen von MDCK-Zysten nach 15 Tagen im PVA-Gel; und
- Fig. 3: die Ergebnisse der Kultivierung von mesenchymalen Stammzellen in PVA und BSA-Gelen (Calcein/Propidiumjodid-Färbung).

### Beispiel 1: Herstellung von maleimidfunktionalisierter BSA

250 mg BSA (Sigma-Aldrich, Kat.-Nr. A7030) wurden in 5 ml 1M Na-Borat (pH 8,2) gelöst. Diese Lösung wurde mit 6 mg N-Maleoyl-β-Alanin (Sigma-Aldrich Kat.-Nr 63285) versetzt und für 2 Stunden bei Raumtemperatur inkubiert. Weiterhin wurden 130 mg N-Hydroxysuccinimid in 730 µl Acetonitril gelöst. Danach wurden 96 mg N-Maleoyl-β-Alanin in 570 µl der N-Hydroxysuccinimid-Lösung gelöst. Zu dieser Lösung wurden 80 µl Acetonitril und 80 µl Diisopropylcarbodiimid gegeben. Nach einer 5-minütigen Inkubation bei Raumtemperatur wurde der Ansatz für 5 min zentrifugiert. Der Überstand wurde tropfenweise zu der BSA-Lösung unter ständigem Rühren zugegeben. Nach Inkubation von 45 min bei Raumtemperatur wurde der Ansatz viermal gegen 500 ml PBS auf Eis dialysiert und anschließend durch Ultrafiltration auf ein Volumen von 4 ml konzentriert.

### Beispiel 2: Herstellung von maleimidfunktionalisiertem PVA

120 mg Carbonyldiimidazol wurden in 3 ml 4% (w/v) PVA in DMSO (Sigma, Kat.-Nr P8136) gelöst und für 3 hr bei Raumtemperatur inkubiert. Diese Lösung wurde danach zu 420 µl Ethylendiamin unter Rühren zugetropft und für 2 Tage bei Raumtemperatur in einem geschlossenen Gefäß gerührt. Aminiertes PVA wurde durch Zugabe von 30 ml Ethanol/Diethylether (1:5) präzipitiert und durch Zentrifugation pelletiert. Das Pellet wurde in 3 ml PBS gelöst und durch Zugabe von 2,5 M HCl auf pH 6 gebracht. Danach wurde die Lösung dreimal gegen 1 Liter 100 mM Na-Carbonat (pH 8,2) dialysiert. Zur Funktionalisierung des aminierten PVA wurden zunächst 75 mg N-Maleoyl-β-Alanin in 1029 µl 1M N-Hydroxysuccinimid in Acetonitril gelöst und durch Zugabe von 125 µl Diisopropylcarbodiimid aktiviert. Nach 10 min bei Raumtemperatur wurde der Ansatz für 5 min zentrifugiert und der Überstand wurde tropfenweise zu der gerührten Amin-PVA-Lösung gegeben. Nach 1 hr bei Raumtemperatur wurde der Ansatz 10 min bei 3000 rpm zentrifugiert und der Überstand wurde dreimal gegen 600 ml PBS dialysiert. Danach wurde der Ansatz durch Ultrafiltration auf 3,5 ml eingeengt.

Statt PVA kann auch Dextran durch Maleimid- oder Thiolgruppen funktionalisiert werden. Zur Herstellung von thiolfunktionalisiertem Dextran wurden beispielsweise zunächst 1 g Dextran (Serva, Kat. Nr. 18690) in 10 ml wasserfreiem Dimethylsulfoxid gelöst. In dieser Lösung wurden zusätzlich 100 mg Carbonyldiimidazol gelöst. Nach 2 Stunden Inkubation bei Raumtemperatur wurden 1,12 g Cystamin x 2 HCl und 1,5 ml Pyridin zugegeben. Zur vollständigen Auflösung des Diamins wurde der Ansatz einige Minuten im Ultraschallbad behandelt. Nach einer zweitägigen Inkubation wurde der Ansatz dreimal gegen 1 Liter H₂O dialysiert (regenerierte Zellulosemembran, MWCO 3000). Während der Dialyse wurde der Säuregehalt durch Zugabe von HCl auf pH 6 eingestellt. Anschließend wurden im Dialysat 347 mg Dithiothreitol gelöst. Modifiziertes Dextran wurde sofort danach von niedermolekularen Komponenten durch Gelfiltrationschromatografie mit Sephadex G25 (HiPrep™ 26/10 Desalting Column (GE Healthcare/Life Sciences) mit 5 mM Na-Phosphat (pH 5) und einer Flussrate von 10 ml/min abgetrennt. Dextran enthaltende Fraktionen wurden vereinigt (36 ml) und anschließend durch Dialyse zunächst gegen 1 Liter 1 mM HCl, und anschließend gegen 1 Liter 0,5 mM HCl unter Stickstoffatmosphäre weiter gereinigt. Zum Schluss wurde der Ansatz durch Lyophilisation auf 9 ml eingeengt und durch Zentrifugation von ungelöstem Material abgetrennt.

### Beispiel 3: Kopplung von Peptiden und Gelbildung

Bindung von Peptiden an Maleimid-BSA: 10 µl Maleimid-BSA wurden mit unterschiedlichen Mengen Peptid HW1 (Acetyl-KGLQGCGLQGK-OH; SEQ ID Nr. 1) oder HW2 (Acetyl-KGGLQGGK-OH; SEQ ID Nr. 2) bei Raumtemperatur für mindestens 10 min in einem Volumen von 12 µl inkubiert. Danach wurde freies von gekoppeltem Peptid durch Gelfiltrationschromatografie aufgetrennt und das Elutionsprofil durch Messung der Absorption bei 214 nm bestimmt. Zur Bestimmung des Einbaus wurden die Chromatogramme integriert. Für HW1 wurde ein Einbau von 93% in Maleimid-BSA und für HW2 ein Einbau von 10% gemessen. Dagegen konnte keine Kopplung von HW1 an unmodifizierte BSA nachgewiesen werden.

Bindung von Peptiden an Maleimid-PVA: 4 µl Maleimid-PVA oder 2 µl 4% PVA wurden mit 100 nmol Peptid HW1 (Acetyl-KGLQGCGLQGK-OH; SEQ ID Nr. 1) oder HW2 (Acetyl-KGGLQGGK-OH; SEQ ID Nr.2) bei Raumtemperatur für mindestens 10 min in einem Volumen von 16 µl inkubiert. Danach wurden 20 µl einer 1:4-Verdünnung durch Gelfiltrationschromatografie aufgetrennt und das Elutionsprofil durch Messung der Absorption bei 214 nm bestimmt. Zur Bestimmung des Einbaus wurden die Chromatogramme integriert. Tabelle 1 zeigt den relativen Anteil nicht gebundener Peptide:

**Tabelle 1: Einbau von Peptiden**

| | | | | | | |
|---|---|---|---|---|---|---|
| Mal-PVA | ✔ | | ✔ | | | ✔ |
| PVA | | | | ✔ | | |
| HW1 | | ✔ | ✔ | ✔ | | |
| HW2 | | | | | ✔ | ✔ |
| Freies Peptid (%) | n/a | 100 | 12 | 96 | 100 | 107 |

Gelbildung: Beide Materialien konnten durch Zugabe eines Vernetzers mit zwei Thiolgruppen zu Gelen umgebildet werden. Tabelle 2 zeigt typische Mengen, die zur Gelbildung in unterschiedlichen Stärken geeignet sind.

**Tabelle 2: Gelbildung**

| | | | | | | |
|---|---|---|---|---|---|---|
| Maleimid-BSA (µl) | 308 | 167 | 178 | | | |
| Maleimid-PVA (µl) | | | | 308 | 168 | 100 |
| PBS(µl) | 0 | 167 | 178 | 0 | 168 | 300 |
| 100 mg/ml Dithio-PEG (3 kDa) (µl) | 92 | 66 | 54 | 92 | 64 | 50 |

### Beispiel 4: Adhäsion von 3T3-Zellen an Gelkomponenten

Auf nitrozellulosebeschichtete Glasobjektträger (NMI Technologietransfer GmbH, Reutlingen) wurden jeweils 1 µl unterschiedlicher Komponenten in einem Druckpuffer (0,5% Trehalose, 1 µg/ml BSA-Tetramethylrhodamin-Konjugat (Invitrogen, Carlsbad, CA; Kat.-Nr.A-23016) in PBS (20 mM Na-Phosphat (pH 7,2), 150 mM NaCl)) aufgetragen. Die Objekträger wurden über Nacht bei Raumtemperatur gelagert und am nächsten Tag mit Blockierlösung (Stabilguard, SurModics, Eden Prairie, MN) eingesprüht um die Flächen zwischen den Komponenten zu blockieren. Danach wurden die Träger zweimal mit PBS gewaschen und mit einer Zellsuspension (10⁵ 3T3-Zellen pro ml) für 2 Stunden geschwenkt. Danach wurde die Zellsuspension entfernt, die Träger wurden mit Comassie-Färbelösung behandelt und fotografiert.

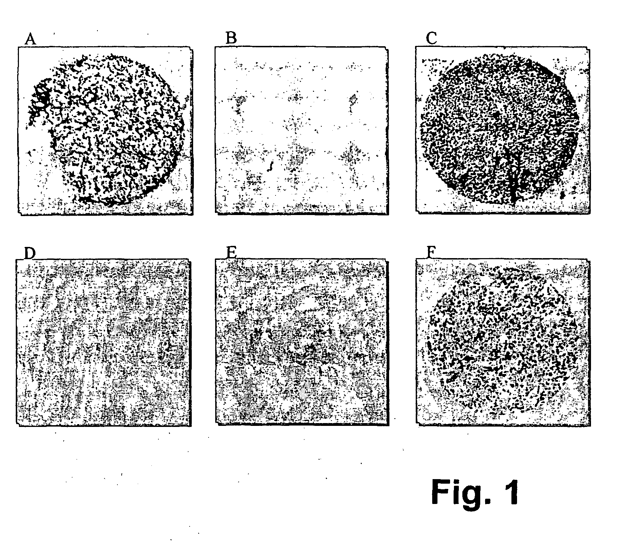

In Fig. 1 sind die Ergebnisse der Adhäsion von 3T3-Zellen auf Gelkomponenten gezeigt, wobei in A 200 µg/ml Fibronektin eingesetzt wurden, in B eine 1:5-verdünnte Maleimid-PVA-Lösung, in C 1 µl einer Lösung bestehend aus 2 µl Maleimid-PVA, 0,5 µl HW9 (FITC-β-Ala-GCGYGRGDSPGSGC; SEQ ID Nr. 3 mit Fluoresceingruppe vor dem β-Alanin; 50 mM), 7,5 µl PBS, in D 20 mg/ml Di-Thio-PEG (3 kDa; Rapp-Polymere Tübingen; Kat.- Nr. 11 3000-40), in E 1:10-verdünnte Maleimid-BSA, und in F 1 µl einer Lösung bestehend aus 2 µl Maleimid-BSA, 0,38 µl HW9 (FITC-β-Ala-GCGYGRGDSPGSGC; SEQ ID Nr. 3 mit Fluoresceingruppe vor dem β-Alanin; 50 mM), 7,62 µl PBS.

### Beispiel 5: Kultivierung von MDCK-Zellen in PVA-Gel

MDCK-Zellen (eine Epithelzelllinie aus der Hundeniere) wurden in einer Dichte von 1 Mio Zellen/ml in PVA-Gele eingesät. (Zusammensetzung des Gels: Maleimid-PVA: 10µl, PBS: 6µl, MDCK-Zellen: 10⁴ Zellen in 10 µl PBS, Vernetzer (Dithio-PEG, 3 kDa; 100 mg/ml): 4 µl). Das Gesamtvolumen des einzelnen Gels betrug hierbei 30 µl, wobei die 4 µl Vernetzer in das Zellkulturgefäß (Nalge Nunc Int., LAB-TEK II, Chambered Coverglass - Borosilicate) vorgelegt wurden. Erst nach der Fertigstellung der restlichen Gelmischung, die auch die Zellen enthielt, wurde der Vernetzer durch 3-maliges auf-und-ab pipettieren mit den Gelkomponenten vermischt. Innerhalb von ca. 10 Sekunden bildete sich darauf hin ein Gel. Das Gel wurde mit 400 µl Kulturmedium (DMEM high Glucose, 4 mM Glutamin, 1% Penicillin/Streptomycin und 10% Fötales Kälberserum) überschichtet und bei 5% CO₂ und 37°C in feuchter Atmosphäre inkubiert. Nach 7 und 15 Tagen Kultivierung der Zellen im PVA-Gel wurde eine Aktinfärbung (TRITC-Phalloidin [0,3 µg/ml], Sigma) und Kernfärbung (Syto Green [167 nM], Molecular Probes) zur Darstellung der MDCK-Zellen durchgeführt.

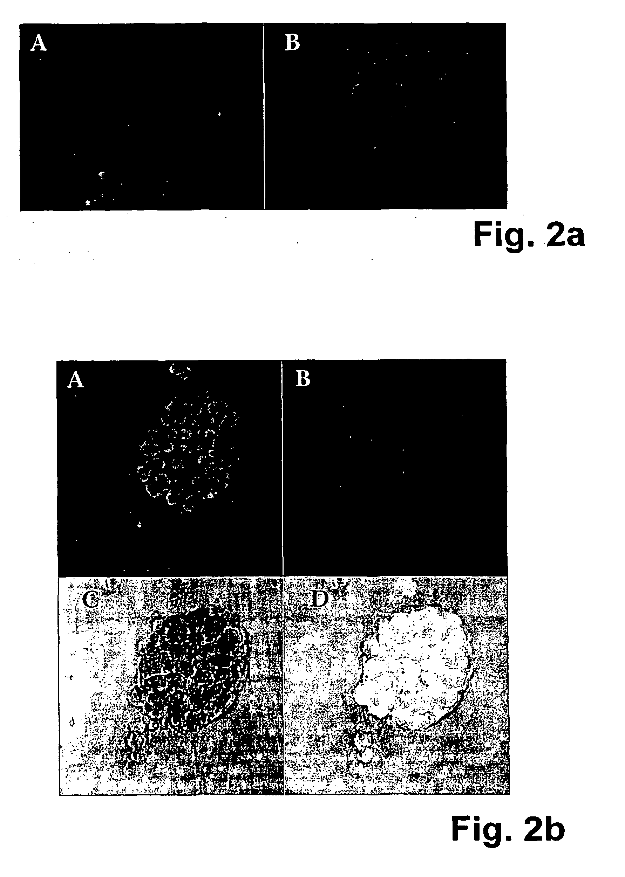

In Fig. 2a und 2b sind konfokale Laser Scanning Mikroskopie-Aufnahmen von MDCK-Zysten nach 15 Tagen im PVA-Gel gezeigt. Bei der gezeigten MDCK-Zyste in Abbildung 2 lässt die Fluoreszenzmarkierung des Aktinzytoskeletts eine Polarität der Zellen mit apikaler Zone in Richtung Lumen und basolateraler Zone in Richtung PVA-Matrix vermuten. Dabei ist in Fig. 2a der optische Schnitt durch eine MDCK-Zyste in PVA-Gel zur Darstellung des Lumens (konfokale Laser-Scanning Mikroskopie) gezeigt: A: Kernfärbung mit Syto Green. B: Aktinfärbung mit TRITC-Phalloidin. In Fig. 2b ist der optische Schnitt im äußeren Bereich durch eine MDCK-Zyste in PVA-Gel gezeigt, wobei das Lumen nicht sichtbar ist: A: Kernfärbung mit Syto Green. B: Aktinfärbung mit TRITC-Phalloidin. C: Phasenkontrastaufnahme. D: Overlay von A, B, und C.

### Beispiel 6: Kultivierung von mesenchymalen Stammzellen in PVA- und BSA-Gelen

2D-Kultivierung der Zellen: Humane mesenchymale Stammzellen wurden mit Standardmethoden in Gewebekulturflaschen (T75-Flaschen, Greiner) kultiviert, durch Trypsinlösung von der Oberfläche abgelöst, pelletiert, in MSC-Medium aufgenommen und gezählt.

3D-Kultivierung: Zur Einbettung von Zellen in BSA-Gelen wurden 21,6 µl Maleimid-BSA und 19 µl Zellsuspension (50000 Zellen) in eine Vertiefung einer Mikrotiterplatte platziert und durch Einmischung von 9,4 µl Vernetzungslösung (100 mg/ml Dithio-PEG, 3000 Da) vernetzt. Zur Einbettung in PVA wurden 15,1 µl Maleimid-PVA und 28,7 µl Zellsuspension (50000 Zellen) in eine Vertiefung einer Mikrotiterplatte platziert und durch Einmischung von 6,2 µl Vemetzungslösung vernetzt. Nach ca. 5 min wurden die Gelpräparate mit 250 µl Medium überschichtet und für 6 Tage bei 37°C kultiviert. Es wurde eine Calcein-Propidiumjodidfärbung durchgeführt und die Gele wurden in einem Fluoreszenzmikroskop fotografiert. Dabei zeigte sich, dass in beiden Gelen der überwiegende Anteil der Zellen (>95%) auch nach 6-tägiger Inkubation noch am Leben war.

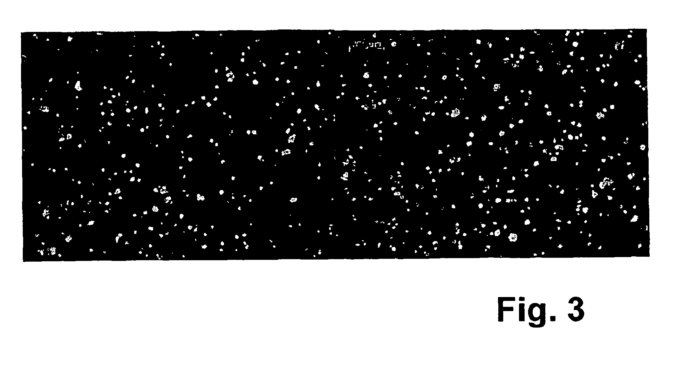

In Fig. 3 sind die Ergebnisse der Calcein/Propidiumjodid-Färbung von mesenchymalen Stammzellen nach 6-tägiger 3-D-Kultivierung gezeigt: A: Zellen in BSA-Gel. B: Zellen in PVA-Gel.

### Beispiel 7: Beispiele für Zellkulturen in BSA- und PVA-Gelen

Es wurden mehrere primäre Zellpräparationen sowie Zelllinien in PVA- und BSA Gelen erfolgreich kultiviert. Tabelle 3 gibt eine Überblick über diese Versuche.

**Tabelle 3: 3D-Zellkultur mit unterschiedlichen Zellarten**

| Zellart | Geltyp | Kultivierungsdauer (Tage) | Assays | Ergebnisse |
|---|---|---|---|---|
| Humane Chondrozyten aus Osteoarthritischem Knorpel | PVA | 16 | Calcein, Propidiumjodid | >80% Überleben |
| Humane Chondrozyten aus Osteoarthritischem Knorpel | BSA | 16 | Calcein, Propidiumjodid | >90% Überleben |
| Maus MC3T3-Zelllinie | PVA | 3 | Calcein, Propidiumjodid | >80% Überleben |
| Maus MC3T3-Zelllinie | BSA | 3 | Calcein, Propidiumjodid | >70% Überleben |
| Humane mesenchymale Stammzellen | PVA | 6 | Calcein, Propidiumjodid | >95% Überleben |
| Humane mesenchymale Stammzellen | BSA | 6 | Calcein, Propidiumjodid | >95% Überleben |
| MDCK | BSA | 6 | Phasenkontrast-Mikroskopie | Bildung von Zysten |
| NIH 3T3 | PVA | 17 | Calcein, Propidiumjodid | >5% Überteben, Bildung von Aggregaten (Größe bis zu 60µm) |
| MDCK | PVA | 17 | Calcein, Propidiumjodid | >50% Überleben, Bildung von Zysten (Größe bis zu 100µm) |
| MDCK | PVA | 7 und 15 | Darstellung der Polaritäl von Zysten durch Actinfärbung | Apikale und basolaterale Zonen darstellbar. Bei den untersuchten Cysten beide Potaritäten gefunden: apikal sowohl in Richtung Lumen als auch in Richtung Matrix. |

Aus den oben aufgeführten Ergebnissen mit den getesteten Biomaterialien zeigt sich, dass sowohl die eingesetzten Polymere, als auch die verwendeten Peptide herausragende Beispiele für die erfindungsgemäßen Biomaterialien sind.

## Patentansprüche

1. Verwendung eines Biomaterials als dreidimensionales Hydrogel für die Kultivierung von Zellen oder von Gewebe, wobei das Biomaterial auf einem polymeren Träger basiert, der zumindest ein vernetzbares hydrophiles Polymer enthält, **dadurch gekennzeichnet, dass** das Polymer mit Maleimid-Gruppen funktionalisiert ist, und dass das Polymer ausgewählt ist aus Polyvinylalkohol und Serumalbumin, oder Mischungen davon und wobei das funktionalisierte Polymer durch Zugabe eines Vernetzers zu dem Polymer vernetzt wird.

2. Verwendung nach Anspruch 1, **dadurch gekennzeichnet, dass** das Polymer in Anwesenheit von Zellen vernetzbar ist.

3. Verwendung nach Anspruch 1 oder 2, **dadurch gekennzeichnet, dass** es ferner zumindest einen Biofaktor aufweist.

4. Verwendung nach Anspruch 3, **dadurch gekennzeichnet, dass** der Biofaktor zumindest eine Thiolgruppe aufweist, über welche die Biofaktoren an Maleimidgruppen des Polymers gebunden sind.

5. Verwendung nach einem der Ansprüche 3 oder 4, **dadurch gekennzeichnet, dass** der Biofaktor ausgewählt ist aus Peptiden, Proteinen, Nukleinsäuren, organische Wirkstoffe, Apoptose-induzierende Wirkstoffe, adhäsionsvermittelnde Wirkstoffe, wachstumshemmende Wirkstoffe, entzündungshemmende Wirkstoffe, Rezeptoragonisten- und Rezeptorantagonisten.

6. Verwendung nach Anspruch 5, **dadurch gekennzeichnet, dass** der Biofaktor ausgewählt ist aus Proteinen der extrazellulären Matrix, Proteinen der Zelloberfläche, Antikörper, Wachstumsfaktoren, Zucker, Lektine, Kohlenhydrate, Zytokine, DNA, RNA, siRNA, Aptamere, sowie bindungs- oder wirkungsrelevanten Fragmenten davon, oder Mischungen davon.

7. Verwendung nach einem der Ansprüche 3 bis 6, **dadurch gekennzeichnet, dass** das Polymer über an Biofaktoren gebundene Zellen vernetzbar ist.

8. Verwendung nach einem der Ansprüche 2 bis 7, **dadurch gekennzeichnet, dass** die Zellen Säugetierzellen sind.

9. Dreidimensionales Hydrogel zur Kultivierung von Zellen oder von Gewebe, wobei das Hydrogel auf einem polymeren Träger basiert, der zumindest ein vernetzbares hydrophiles Polymer enthält, **dadurch gekennzeichnet, dass** das Polymer mit Maleimid-Gruppen funktionalisiert ist und ausgewählt ist aus Polyvinylalkohol und Serumalbumin, oder Mischungen davon, und durch Zugabe eines Vernetzers vernetzt wird.

10. Verfahren zur Herstellung eines Biomaterials als Hydrogel für dreidimensionale Zellkulturenanwendungen, das die folgenden Schritte aufweist:
a) Bereitstellen eines hydrophilen Polymers, das ausgewählt ist aus Serumalbumin und Polyvinylalkohol, oder Mischungen davon,
b) Funktionalisieren des Polymers aus Schritt a) mit Maleimid, und
c) Hinzufügen eines Vernetzers zum Vernetzen des in Schritt b) funktionalisierten Polymers.

11. Verfahren nach Anspruch 10, **dadurch gekennzeichnet, dass** es ferner den Schritt a)' aufweist:
a)' Aminierung des Polymers.

12. Verfahren nach Anspruch 10 oder 11, **dadurch gekennzeichnet, dass** es ferner nach Schritt b) den Schritt b)' aufweist:
b)' Hinzufügen von und Inkubieren mit Biofaktoren und/oder Zellen, insbesondere Säugetierzellen.

13. Verfahren nach Anspruch 12, **dadurch gekennzeichnet, dass** der Biofaktor ausgewählt ist aus Peptiden, Proteinen, Nukleinsäuren, organische Wirkstoffe, Apoptose-induzierende Wirkstoffe, adhäsionsvermittelnde Wirkstoffe, wachstumshemmende Wirkstoffe, entzündungshemmende Wirkstoffe, Rezeptoragonisten- und Rezeptorantagonisten, und insbesondere aus Proteinen der extrazellulären Matrix, Proteinen der Zelloberfläche, Antikörper, Wachstumsfaktoren, Zucker, Lektine, Kohlenhydrate, Zytokine, DNA, RNA, siRNA, Aptamere, sowie bindungs- oder wirkungsrelevanten Fragmenten davon, oder Mischungen davon.

14. Verfahren nach Anspruch 13, **dadurch gekennzeichnet, dass** der Biofaktor ausgewählt ist aus Proteinen der extrazellulären Matrix, Wachstumsfaktoren, bindungs- oder wirkungsrelevanten Fragmenten davon, oder Mischung davon.

15. Verfahren nach einem der Ansprüche 10 bis 14, **dadurch gekennzeichnet, dass** der Vernetzer in Schritt c) ausgewählt ist aus thiolfunktionalisiertem PEG.

16. Verfahren nach einem der Ansprüche 10 bis 15, **dadurch gekennzeichnet, dass** ein Hydrogel nach Anspruch 9 hergestellt wird.

## Claims

1. Use of a biomaterial as a three-dimensional hydrogel for the cultivation of cells or tissue, wherein the biomaterial is based on a polymeric carrier, which contains at least one crosslinkable hydrophilic polymer, **characterized in that** the polymer is functionalized with maleimide groups, and that the polymer is selected from polyvinyl alcohol and serum albumin, or mixtures thereof, and wherein the functionalized polymer is crosslinked by addition of a crosslinking agent.

2. The use as claimed in claim 1, **characterized in that** the polymer is crosslinkable in the presence of cells.

3. The use as claimed in one of claims 1 or 2, **characterized in that** the biomaterial additionally has at least one biofactor.

4. The use as claimed in claim 3, **characterized in that** the biofactor has at least one thiol group, via which the biofactors are bound to maleimide groups of the polymer.

5. The use as claimed in claim 3 or 4, **characterized in that** the biofactor is selected from peptides, proteins, nucleic acids, organic active substances, apoptosis-inducing active substances, adhesion-promoting active substances, growth-inhibiting active substances, antiinflammatory active substances, receptor agonists and receptor antagonists.

6. The use as claimed in claim 5, **characterized in that** the biofactor is selected from proteins of the extracellular matrix, cell surface proteins, antibodies, growth factors, sugars, lectins, carbohydrates, cytokines, DNA, RNA, siRNA, aptamers, and fragments thereof that are relevant to binding or action, or mixtures thereof.

7. The use as claimed in one of claims 3 to 6, **characterized in that** the polymer is crosslinkable via cells bound to biofactors.

8. The use as claimed in one of claims 2 to 7, **characterized in that** the cells are mammalian cells.

9. A three-dimensional hydrogel for the cultivation of cells or tissue, wherein the biomaterial is based on a polymeric carrier, which contains at least one crosslinkable hydrophilic polymer, **characterized in that** the polymer is functionalized with maleimide groups, and is selected from polyvinyl alcohol and serum albumin, or mixtures thereof, and is crosslinked by addition of a crosslinking agent

10. A method of production of a biomaterial for cell culture applications, which has the following steps:
a) preparation of a hydrophilic polymer, which is selected from serum albumin and polyvinyl alcohol, or mixtures thereof,
b) functionalization of the polymer from step a) with maleimide, and
c) addition of a crosslinking agent for crosslinking the polymer functionalized in step b).

11. The method as claimed in claim 10, **characterized in that** it additionally has step a)':
a)' amination of the polymer.

12. The method as claimed in claim 10 or 11, **characterized in that**, after step b), it additionally has step b)':
b)' addition of and incubation with biofactors and/or cells, especially mammalian cells.

13. The method as claimed in claim 12, **characterized in that** the biofactor is selected from peptides, proteins, nucleic acids, organic active substances, apoptosis-inducing active substances, adhesion-promoting active substances, growth-inhibiting active substances, antiinflammatory active substances, receptor agonists and receptor antagonists, and in particular from proteins of the extracellular matrix, cell surface proteins, antibodies, growth factors, sugars, lectins, carbohydrates, cytokines, DNA, RNA, siRNA, aptamers, and fragments thereof that are relevant to binding or action, or mixtures thereof.

14. The method as claimed in claim 13, **characterized in that** the biofactor is selected from proteins of the extracellular matrix, growth factors, fragments thereof that are relevant to binding or action, or mixtures thereof.

15. The method as claimed in one of claims 10 to 14, **characterized in that** the crosslinking agent in step c) is selected from thiol-functionalized PEG.

16. The method as claimed in one of claims 10 to 16, **characterized in that** the biomaterial as claimed in claim 9 is produced.

## Revendications

1. Utilisation d'un biomatériau en guise d'hydrogel en trois dimensions pour la culture de cellules ou de tissu, le biomatériau étant à base d'un support polymère qui contient au moins un polymère hydrophile réticulable, **caractérisée en ce que** le polymère est fonctionnalisé avec des groupes de maléimide, et **en ce que** le polymère est sélectionné parmi l'alcool polyvinylique et l'albumine sérique ou des mélanges de ceux-ci et le polymère fonctionnalisé étant réticulé par ajout d'un agent réticulant au polymère.

2. Utilisation selon la revendication 1, **caractérisée en ce que** le polymère est réticulable en présence de cellules.

3. Utilisation selon la revendication 1 ou 2, **caractérisée en ce qu'**il présente de plus au moins un biofacteur.

4. Utilisation selon la revendication 3, **caractérisée en ce que** le biofacteur présente au moins un groupe de thiol, par lequel les biofacteurs sont liés aux groupes de maléimide du polymère.

5. Utilisation selon l'une quelconque des revendications 3 ou 4, **caractérisée en ce que** le biofacteur est sélectionné parmi les peptides, les protéines, les acides nucléiques, les substances actives organiques, les substances actives induisant l'apoptose, les substances actives transmettant l'adhésion, les substances actives empêchant la croissance, les substances actives empêchant l'inflammation, les agonistes de récepteur et les antagonistes de récepteur.

6. Utilisation selon la revendication 5, **caractérisée en ce que** le biofacteur est sélectionné parmi les protéines de la matrice extracellulaire, les protéines de la surface cellulaire, les anticorps, les facteurs de croissance, le sucre, les lectines, les glucides, les cytokines, l'ADN, l'ARN, les pARNi, les aptamères ainsi que des fragments importants pour la liaison ou l'action de ceux-ci ou mélanges de ceux-ci.

7. Utilisation selon l'une quelconque des revendications 3 à 6, **caractérisée en ce que** le polymère est réticulable par des cellules liées aux biofacteurs.

8. Utilisation selon l'une quelconque des revendications 2 à 7, **caractérisée en ce que** les cellules sont des cellules de mammifère.

9. Hydrogel en trois dimensions pour la culture de cellules ou de tissu, l'hydrogel étant à base d'un support polymère qui contient au moins un polymère hydrophile réticulable, **caractérisé en ce que** le polymère est fonctionnalisé avec des groupes de maléimide et est sélectionné parmi l'alcool polyvinylique et l'albumine sérique ou des mélanges de ceux-ci et est réticulé par ajout d'un agent réticulant.

10. Procédé de fabrication d'un biomatériau servant d'hydrogel pour des applications de culture de cellule en trois dimensions, qui présente les étapes suivantes :
a) mise à disposition d'un polymère hydrophile qui est sélectionné parmi l'albumine sérique et l'alcool polyvinylique ou des mélanges de ceux-ci,
b) fonctionnalisation du polymère de l'étape a) avec un maléimide, et
c) ajout d'un agent réticulant pour la réticulation du polymère fonctionnalisé à l'état b).

11. Procédé selon la revendication 10, **caractérisé en ce qu'**il présente de plus l'étape a)' :
a)' amination du polymère.

12. Procédé selon la revendication 10 ou 11, **caractérisée en ce qu'**il présente après l'étape b) l'étape b)' :
b)' ajout et incubation avec des biofacteurs et/ou des cellules, en particulier des cellules de mammifère.

13. Procédé selon la revendication 12, **caractérisé en ce que** le biofacteur est sélectionné parmi les peptides, les protéines, les acides nucléiques, les substances actives organiques, les substances actives induisant l'apoptose, les substances actives transmettant l'adhésion, les substances actives empêchant la croissance, les substances actives empêchant l'inflammation, les agonistes de récepteur et les antagonistes de récepteur, et en particulier les protéines de la matrice extracellulaire, les protéines de la surface cellulaire, les anticorps, les facteurs de croissance, le sucre, les lectines, les glucides, les cytokines, l'ADN, l'ARN, les pARNi, les aptamères ainsi que des fragments importants pour la liaison ou l'action de ceux-ci ou mélanges de ceux-ci.

14. Procédé selon la revendication 13, **caractérisé en ce que** le biofacteur est sélectionné parmi les protéines de la matrice extracellulaire, les facteurs de croissance, des fragments importants pour la liaison ou l'action de ceux-ci ou des mélanges de ceux-ci.

15. Procédé selon l'une quelconque des revendications 10 à 14, **caractérisé en ce que** l'agent réticulant de l'étape c) est sélectionné parmi le polyéthylèneglycol fonctionnalisé avec le thiol.

16. Procédé selon l'une quelconque des revendications 10 à 15, **caractérisé en ce qu'**un hydrogel selon la revendication 9 est fabriqué.


### SEQUENZ PROTOKOLL

<110> NMI Naturwissenschftliches und Medizinisches Institut an der Universität Tübingen
<120> Biomaterial basierend auf einem hydrohphilen polymeren Träger
<130> 3605P139
<160> 3
<170> PatentIn version 3.1
<210> 1<211> 11<212> PRT<213> Artificial Sequence
<220>
   <223> synthesized peptide
<220>
   <221> MOD_RES<222> (1)..(1)<223> ACETYLATION
<400> 1
   Lys Gly Leu Gln Gly Cys Gly Leu Gln Gly Lys
   1 5 10
<210> 2<211> 8<212> PRT<213> Artificial Sequence
<220>
   <223> synthesized peptide
<220><221> MOD_RES<222> (1)..(1)<223> ACETYLATION
<400> 2
   Lys Gly Gly Leu Gln Gly Gly Lys
   1 5
<210> 3<211> 15<212> PRT<213> Artificial Sequence
<220>
   <223> synthesized peptide
<220><221> MOD_RES<222> (1)..(1)<223> bAla
<400> 3
   Ala Gly Cys Gly Tyr Gly Arg Gly Asp Ser Pro Gly Ser Gly Cys
   1 5 10 15